# NEUE EUROPÄISCHE PATENTSCHRIFT

(11) **EP 1 032 446 B2**
(45) Veröffentlichungstag und Bekanntmachung des Hinweises auf die Entscheidung über den Einspruch: **27.11.2013**
(45) Hinweis auf die Patenterteilung: 06.05.2004
(21) Anmeldenummer: 98962331.9
(22) Anmeldetag: 11.11.1998
(51) Int. Cl.: A61M 5/46, A61M 5/32

(54) **NADELANORDNUNG**
NEEDLE ARRANGEMENT
CONFIGURATION D'AIGUILLE

(30) Priorität: 19.11.1997 DE 29720513 U
(43) Veröffentlichungstag der Anmeldung: 06.09.2000
(73) Patentinhaber: Sanofi-Aventis Deutschland GmbH, 65929 Frankfurt am Main (DE)
(72) Erfinder: GABRIEL, Jochen, D-70192 Stuttgart (DE); POLZIN, Ulf, D-70771 Leinfelden (DE)
(74) Vertreter: KEIL & SCHAAFHAUSEN Patentanwälte
(86) Internationale Anmeldenummer: PCT/EP1998/007230
(87) Internationale Veröffentlichungsnummer: WO 1999/025402

(56) Entgegenhaltungen:
- EP-A- 0 182 682
- EP-A2- 0 279 583
- WO-A1-93/16746
- DE-U- 8 909 799
- FR-A- 2 700 961
- US-A- 2 677 373
- US-A- 2 876 770
- US-A- 5 292 314
- US-A- 5 429 612
- US-A- 5 578 014
- US-A- 5 658 256

## Beschreibung

Die Erfindung betrifft eine Nadelanordnung für ein Injektionsgerät.

Eine derartige Nadelanordnung ist bekannt aus der EP-A1-0 749 758 oder dem DE-U1-8 909 799.8. Bei ihr wird eine Kanüle verwendet, die an einem Kanülenhalter befestigt ist. Letzterer wird auf ein Außengewinde am proximalen Ende des Injektionsgeräts aufgeschraubt. (Proximal = Seite des Injektionsgeräts mit der Nadel). Über diese Kanüle wird anschließend eine spezielle Vorrichtung geschoben, welche die Kanüle für den Benutzer unsichtbar macht, um ihm die Angst vor einer Injektion zu nehmen.

Femer kennt man aus der US-A-5 429 612 eine Nadelanorclnung mit einer Nadel (Kanüle), welche an einem Kanülenträger befestigt ist. Auf diesen ist ein kappenartiges Teil aufgeschoben, auf dem seinerseits eine Nadelschutzkappe verschiebbar angeordnet ist. Zwischen dem kappenartigen Teil und der Nadelschutzkappe befindet sich eine Druckfeder. Während einer Injektion wird diese Druckfeder zusammengepresst, und wenn sie nach einer Injektion die Nadelschutzkappe verschiebt, wird letztere durch einen Sprengring in der Lage festgehalten, in der sie die Kanüle voll umgibt. Dadurch wird eine unabsichbiche Verletzung durch die Nadel, oder eine Infektion durch die Nadel, vermieden.

Aus der US-A-5 658 256 kennt man verschiedene Nadelanordnungen, die nach der Injektion eine Kontamination des Benutzers durch die benutzte Nadel verhindem sollen. Hierzu dient eine flexible Hülle, welche sich über die gesamte Nadellänge erstreckt und im Bereich der Nadelspitze durch einen Einsatz verstärkt ist Diese flexible Hülle kann im Bereich der Nadelspitze verschlossen sein und wird dann bei der Injektion von der Nadel durchstochen. Alternativ wird über diese Hülle eine zylindrische Abdeckung geschoben, die im Bereich der Nadelspitze verschlossen ist und die am Kanülenhalter luftdicht befestigt ist.

Aus der WO 93/16746 A ist eine Nadelanordnung für ein Injektionsgerät bekannt, mit einem am proximalen Ende des Injektionsgerätes vorgesehenen Gewinde, mit einem Kanülenträger, an dem eine Kanüle befestigt ist, und der durch ein Gewinde am Kanülenträger zur lösbaren Befestigung am Injektionsgerät ausgebildet ist, mit einer auf dem Kanülenträger etwa parallel zur Längserstreckung der Kanüle verschiebbar angeordneten ersten Kappe, welche an ihrem proximalen Endabschnitt eine Durchtrittsöffnung für die Kanüle aufweist und in ihrer proximalen Endstellung die Kanüle im wesentlichen verdeckt. Zwischen Kanülenträger und verschiebbarer erster Kappe kann nach der Ausführungsform der Fig. 14 bis 17 eine Druckfeder zum Verschieben der ersten Kappe in diese proximale Endstellung angeordnet sein. Die Nadelanordnung umfasst eine als Abdeckkappe und als Montagehilfe zum Aufschrauben der Nadelanordnung auf das Injektionsgerät ausgebildete zweite Kappe, welche die verschiebbare erste Kappe, die Kanüle und den Kanülenträger zumindest teilweise umschließt.

Eine Nadelanordnung ohne eine zweite Kappe ist aus US 5,292,314 bekannt

Weiter offenbart die US 2,677,373 die Verbindung einer Kappe mit einem Kanülenträger. Hier ist der Kanülenträger aber nicht von einer weiteren, verschiebbaren Kappe umgeben.

Es ist eine Aufgabe der Erfindung, eine neue Nadelanordnung für ein Injektionsgerät bereitzustellen.

Nach der Erfindung wird diese Aufgabe gelöst durch den Gegenstand des Patentanspruchs 1, Eine solche Nadelanordnung kann in sehr einfacher Weise bis zur Benutzung steril gehalten werden. Die Abdeckkappe ist als Montagehilfe verwendbar, was die Verwendung durch den Patienten zusätzlich erleichtert. Bevorzugt wird das abnehmbare Verschlussglied als Peelfolie ausgebildet, weil das die Benutzung einfach und sinnfällig macht.

In jedem Fall wird, wenn der Patient die Kanüle vor einer Injektion einsticht, die verschiebbare Kappe entgegen der Kraft der Feder in distaler Richtung verschoben, und beim Herausziehen der Kanüle bewegt sie sich unter der Wirkung der Feder wieder in ihre proximale Endstellung zurück, so daß der Patient während des gesamten Injektionsvorgangs die Kanüle nicht zu sehen bekommt. Durch die lösbare Befestigung am Injektionsgerät kann eine solche Nadelanordnung nach einer Injektion in sehr einfacher Weise durch eine neue, sterile Nadelanordnung ersetzt werden.

Weitere Einzelheiten und vorteilhafte Weiterbildungen der Erfindung ergeben sich aus dem im folgenden beschriebenen und in der Zeichnung dargestellten, in keiner Weise als Einschränkung der Erfindung zu verstehenden Ausführungsbeispiel, sowie aus den Unteransprüchen. Es zeigt:
- Fig. 1: einen Längsschnitt durch eine bevorzugte Ausführungsform einer erfindungsgemäßen Nadelanordnung, in auseinandergezogener und stark vergrößerter Darstellungsweise,
- Fig. 2: eine Darstellung analog Fig. 1, aber im montierten Zustand, wobei die Kanüle von der Anordnung verdeckt ist,
- Fig. 3: eine Darstellung analog Fig. 2, aber bei eingestochener Nadel, wobei die Stichtiefe mit D bezeichnet ist,
- Fig. 4: eine Darstellung analog Fig. 2, wobei zusätzlich eine äußere Abdeckkappe 66 dargestellt ist, welche dazu dient, die Nadelanordnung steril zu umhüllen,
- Fig. 5: eine Darstellung einer fertig konfektionierten Nadelanordnung nach einer bevorzugten Ausführungsform der Erfindung,
- Fig. 6: eine Draufsicht, gesehen in Richtung des Pfeiles VI der Fig. 5,
- Fig. 7: eine Darstellung, welche die Einstellung der Stichtiefe mit Hilfe der äußeren Abdeckkappe 66 zeigt,
- Fig. 8: einen Schnitt, gesehen längs der Linie A-A der Fig. 7,
- Fig. 9: einen Schnitt, gesehen längs der Linie B-B der Fig. 7, und
- Fig. 10: einen Schnitt durch eine Sollbruchstelle für ein Anschlagteil, gesehen längs der Linie C-C der Fig. 7.

In der nachfolgenden Beschreibung werden die Begriffe proximal und distal in der in der Medizin üblichen Weise benutzt, also:

Proximal = dem Patienten zugewandt (die Seite des Injektionsgeräts mit der Nadel).
Distal = vom Patienten abgewandt.

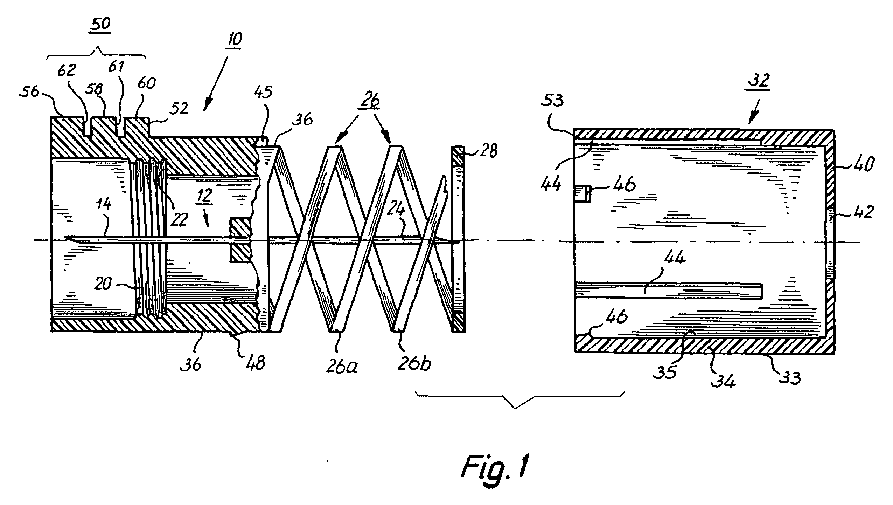

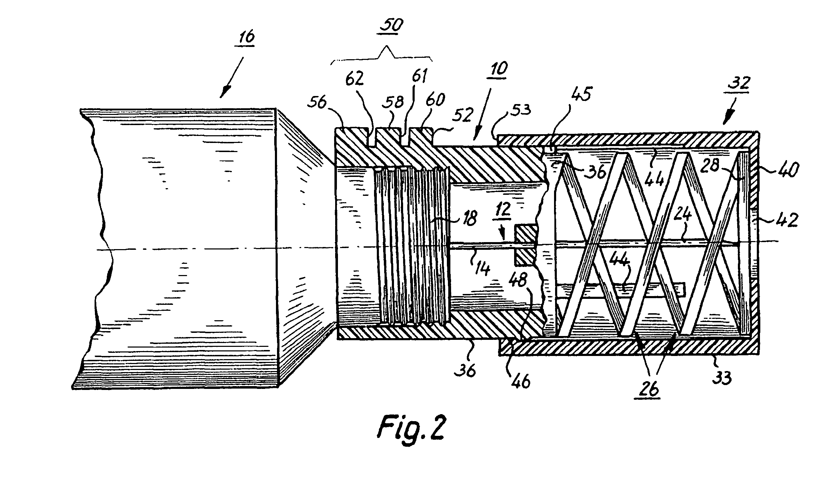

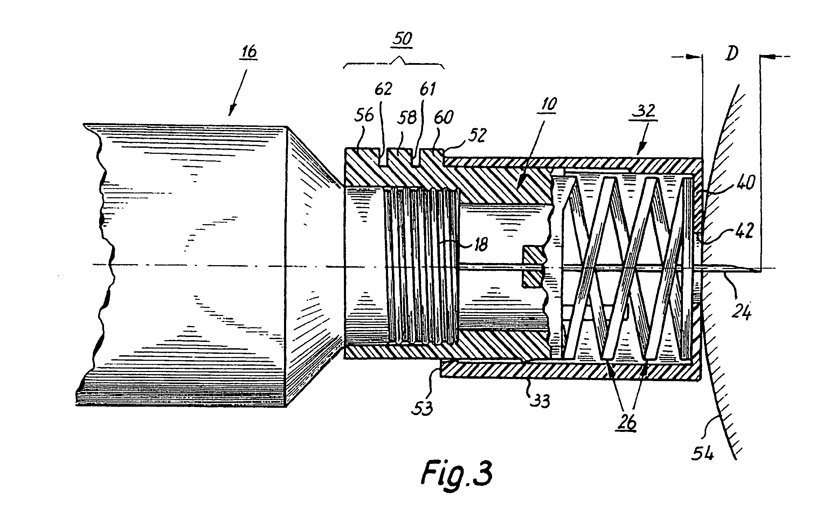

Fig. 1 zeigt links einen Kanülenträger 10 aus einem geeigneten Kunststoff, z.B. Polyethylen. In diesem ist eine Kanüle (Injektionsnadel) 12 befestigt, deren distales Ende 14 zum Durchstoßen der (nicht dargestellten) Gummimembran am Vorratsbehälter eines Injektionsgeräts 16 dient, das in den Fig. 2 und 3 nur schematisch angedeutet ist.

Zum lösbaren Befestigen an einem Außengewinde 18 am proximalen Ende des Injektionsgeräts 16 dient ein Innengewinde 20 des Kanülenträgers 10, das in proximaler Richtung von einer als Anschlag dienenden Schulter 22 begrenzt ist.

Der proximale Abschnitt der Kanüle 12 ist mit 24 bezeichnet. Konzentrisch um ihn herum erstreckt sich bei der Anordnung gemäß Fig. 1 eine Kunststoffeder 26, die mit dem Kanülenträger 10 einstückig ausgebildet sein kann und die hier aus zwei um 180° versetzten Schraubenfedern oder Wendeln 26a, 26b besteht, die an ihrem proximalen Ende jeweils in einen Ring 28 übergehen, mit dem sie ebenfalls einstückig ausgebildet sein können. Alternativ könnte hier auch eine separate Feder verwendet werden, z.B. aus Metall.

Eine erste Hülse oder Kappe 32 hat einen im wesentlichen zylindrischen Abschnitt 34, dessen zylindrische Außenseite mit 33 bezeichnet ist und dessen zylindrische Innenseite 35 zur gleitenden Verschiebung auf dem ebenfalls zylindrischen Umfang 36 des Kanülenträgers 10 ausgebildet ist. Ferner hat die erste Kappe 32 an ihrem proximalen Ende einen Boden 40, in dessen Mitte sich eine Ausnehmung 42 befindet, durch welche bei einer Injektion das proximale Ende 24 der Kanüle 12 durchtreten kann, wie das Fig. 3 zeigt.

Die erste Kappe 32 hat auf ihrer Innenseite 35 insgesamt drei zur axialen Führung, also zur Verdrehsicherung, dienende, am Umfang gleichmäßig verteilte Längsnuten 44, von denen in Fig. 1 nur zwei sichtbar sind. Sie wirken zusammen mit drei hierzu komplementären Vorsprüngen 45 am zylindrischen Außenumfang 36 des Kanülenträgers 10, wie das die Fig. 2 und 3 klar zeigen.

Die erste Kappe 32 hat ferner drei Widerhaken 46 an ihrem Innenumfang 35. Diese Widerhaken sind ebenfalls am Umfang gleichmäßig verteilt und wirken mit drei entsprechenden, komplementären Widerhaken 48 am Außenumfang 36 des Kanülenträgers 10 zusammen, von denen in Fig. 1 nur einer sichtbar ist. Bei der Montage gleiten die Widerhaken 46 über die Widerhaken 48, wodurch die Teile 10 und 32 unlösbar, aber axial verschiebbar, miteinander verbunden werden, wobei die Widerhaken 46, 48 einen Anschlag in proximaler Richtung bilden, wie in Fig. 2 dargestellt, und die Nuten 44 im Zusammenwirken mit den komplementären Vorsprüngen 45 eine Verdrehsicherung für die erste Kappe 32 bilden, so daß diese relativ zum Kanülenträger 10 nicht verdrehbar ist.

Wie die Fig. 1 bis 3 klar zeigen, befindet sich am Außenumfang 36 des Kanülenträgers 10 eine Anschlaganordnung 50, gegen deren proximale Schulter 52 gemäß Fig. 3 die erste Kappe 32 dann mit ihrem distalen Ende 53 zum Anschlag kommt, wenn die Kanüle 12 mit ihrem proximalen Ende 24 in einen nur schematisch angedeuteten Körperteil 54 eingestochen wird.

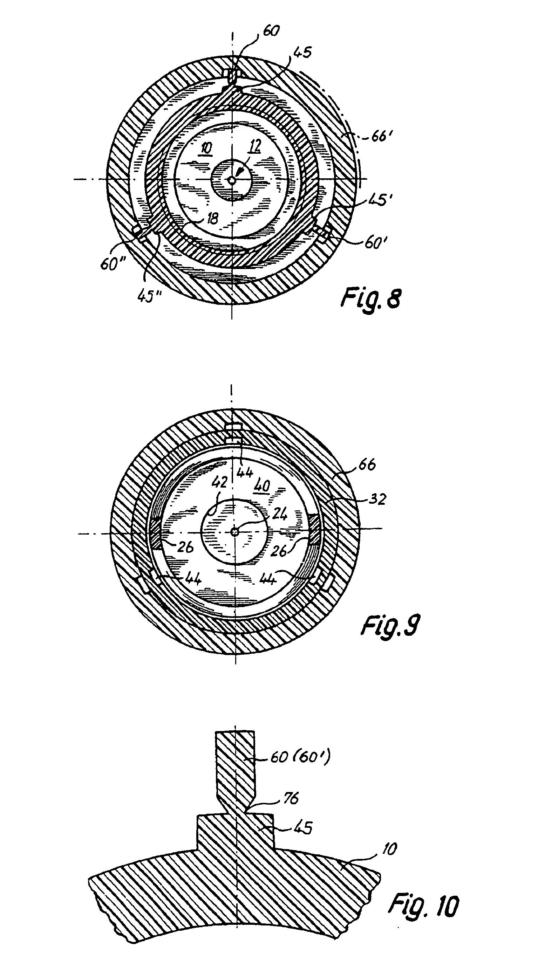

Die Anschlaganordnung 50 weist hier ein distales Anschlagteil 56, ein mittleres Anschlagteil 58, und ein proximales Anschlagteil 60 auf. Zumindest das proximale Anschlagteil 60 und das mittlere Anschlagteil 58 sind jeweils über eine Sollbruchstelle 76 (Fig. 10) mit dem Kanülenträger 10 einstückig verbunden und können folglich durch den Benutzer vom Kanülenträger 10 abgebrochen werden. Dadurch vergrößert sich die Einstichtiefe D (Fig. 3) des proximalen Kanülenteils 24. Man kann also entweder nur das Anschlagteil 60 abbrechen, wobei dann beim Einstechen der Kanüle 12 die erste Kappe 32 gegen eine Schulter 61 zum Anschlag kommt, oder man kann beide Anschlagteile 58 und 60 abbrechen, wobei dann beim Einstechen der Kanüle 12 die erste Kappe 32 gegen eine Schulter 62 zum Anschlag kommt. Im letzteren Fall ergibt sich die maximale Einstichtiefe.

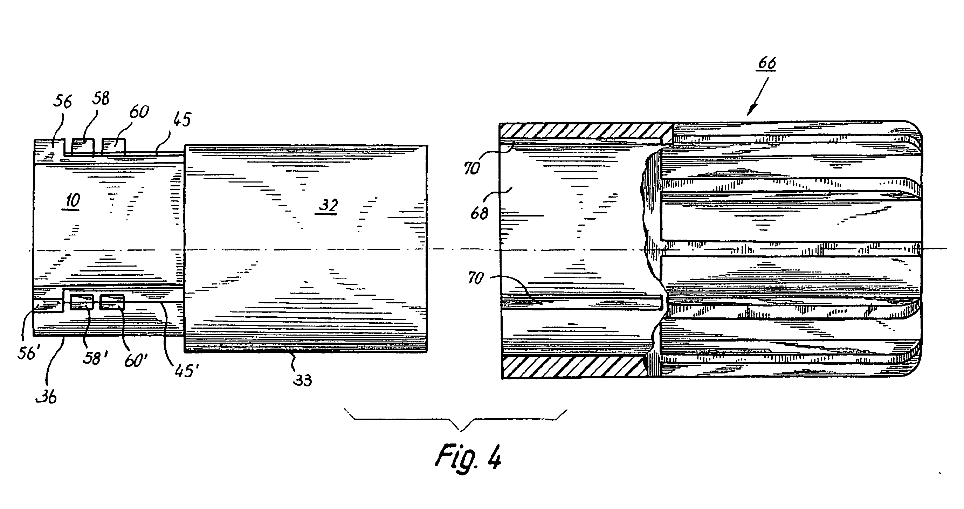

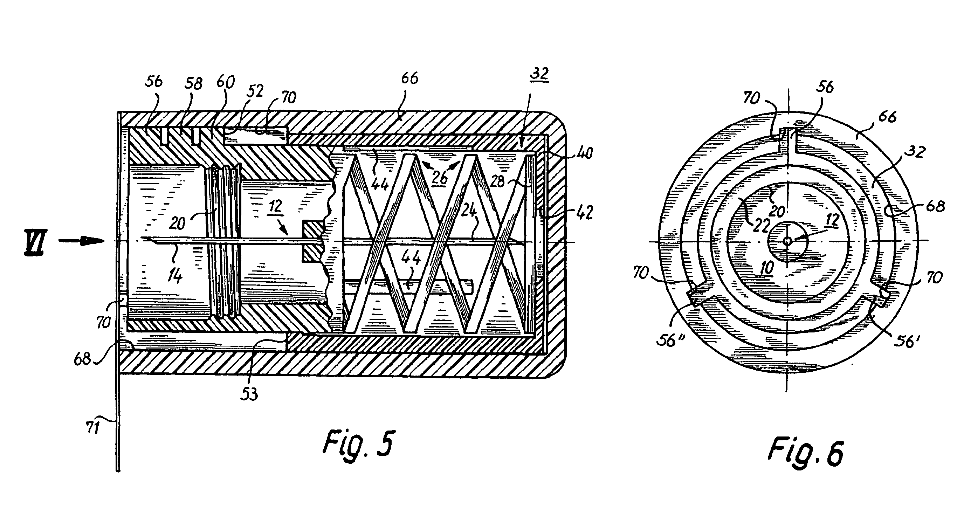

Fig. 4 zeigt links den Kanülenträger 10, an dessen Umfang in gleichmäßigen Abständen von 120° Anschlagteile 56, 58, 60 bzw. 56', 58', 60', 56" etc. angeordnet sind. Fig. 6 zeigt die drei Anschlagteile 56, 56' und 56" in der Draufsicht gemäß dem Pfeil VI der Fig. 5.

Gemäß Fig. 4 ist auch eine äußere Abdeckkappe 66 vorgesehen, welche zur sterilen Abdeckung der Nadelanordnung dient. Die äußere Abdeckkappe 66 ist in Fig. 4 teilweise im Längsschnitt dargestellt, und man erkennt, daß ihre zylindrische Innenausnehmung 68, welche bei der fertigen Nadelanordnung gemäß Fig. 5 und 6 über die zylindrische Außenseite 33 der ersten Kappe 32 geschoben wird, drei Längsnuten 70 aufweist, welche gleichmäßig am Umfang der Innenausnehmung 68 verteilt und so bemessen sind, daß sie über die Anschlagteile 56, 58, 60, 56', 58', 60', 56" etc. geschoben werden können, wie sich das besonders klar aus Fig. 6 ergibt.

Fig. 5 zeigt auch eine Schutzfolie 71, mit der bei der fertigen Nadelanordnung die Öffnung (Fig. 5, links) der äußeren Abdeckkappe 66 steril verschlossen ist. Diese Folie ist aufgeschweißt oder aufgeklebt und wird vor der Benutzung abgerissen. In Fig. 6 ist die Folie 71 nicht dargestellt.

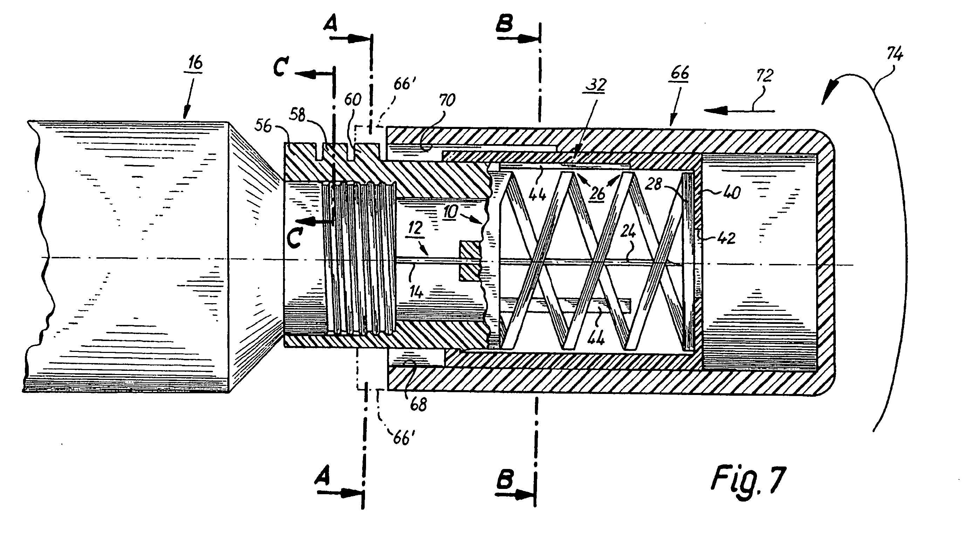

Fig. 7 zeigt, wie die äußere Abdeckkappe 66 auf der ersten Kappe 32 in Richtung des Pfeiles 72 axial verschoben werden kann, wobei sie in eine Lage 66' gelangt, die in Fig. 7 mit strichpunktierten Linien angedeutet und in Fig. 8 im Schnitt dargestellt ist und bei der ihre Längsnuten 70 in Eingriff stehen mit den Anschlagteilen 60, 60', 60". Wird nun die äußere Abdeckkappe 66 in Richtung des in Fig. 7 dargestellten Drehpfeils 74 verdreht, so werden die Anschlagteile 60, 60', 60" entlang ihrer Sollbruchstellen 76 (vgl. Fig. 10) abgebrochen, d.h, die Einstichtiefe D (Fig. 3) wird in der bereits eingehend beschriebenen Weise entsprechend vergrößert. Auf die gleiche Weise kann man auch beide Anschlagteile 58, 60 (dazu 58', 60' etc.) abbrechen und die Einstichtiefe D dadurch noch weiter vergrößern.

Es handelt sich also um eine Nadelanordnung für ein Injektionsgerät 16. Sie hat einen Kanülenträger 10, an dem eine Kanüle 12 befestigt ist und der zur lösbaren Befestigung am Injektionsgerät 16 ausgebildet ist. Die Anordnung hat eine Kappe 32, die auf dem Kanülenträger 10 etwa parallel zur Längserstreckung der Kanüle 12 verschiebbar angeordnet ist, an ihrem proximalen Endabschnitt mit einer Durchtrittsöffnung 42 für die Kanüle 12 versehen ist, und in ihrer proximalen Endstellung die Kanüle 12 im wesentlichen verdeckt. Zwischen Kanülenträger 10 und Kappe 32 ist eine Druckfeder 26 angeordnet zwecks Verschieben der Kappe 32 in ihre proximale Endstellung. Ferner ist eine Abdeckkappe 66 vorgesehen, welche die verschiebbare Kappe 32, die Kanüle 12 und den Kanülenträger 10 umschließt und auf ihrer offenen Seite durch ein abreißbares Verschlußglied 71 steril verschlossen ist. Eine solche Nadelanordnung kann nach einer Injektion leicht ausgetauscht werden. Sie verbessert die Compliance, weil der Patient die Kanüle 12 zu keinem Zeitpunkt sieht. Die Druckfeder kann als Kunststoffeder 26 ausgebildet sein. Sie ist bevorzugt einstückig mit dem Kanülenträger 10, was die Fertigung vereinfacht.

Naturgemäß sind im Rahmen der vorliegenden Erfindung auch sonst vielfache Abwandlungen und Modifikationen möglich.

## Patentansprüche

1. Nadelanordnung für ein Injektionsgerät (16), mit einem am proximalen Ende des Injektionsgerätes (16) vorgesehenen Außengewinde (18), mit einem Kanülenträger (10), an dem eine Kanüle (12) befestigt ist, und der durch ein Innengewinde (20) am Kanülenträger (10) zur lösbaren Befestigung am Injektionsgerät (16) ausgebildet ist, mit einer auf dem Kanülenträger (10) etwa parallel zur Längserstreckung der Kanüle (12) verschiebbar angeordneten ersten Kappe (32), welche an ihrem proximalen Endabschnitt eine Durchtrittsöffnung (42) für die Kanüle (12) aufweist und in ihrer proximalen Endstellung die Kanüle (12) im wesentlichen verdeckt, mit einer zwischen Kanülenträger (10) und verschiebbarer erster Kappe (32) angeordneten Druckfeder (26) zum Verschieben der ersten Kappe (32) in diese proximale Endstellung, wobei die Nadelanordnung eine als Abdeckkappe (66) und als Montagehilfe zum Aufschrauben der Nadelanordnung auf das Injektionsgerät (16) ausgebildete zweite Kappe umfasst, welche die verschiebbare erste Kappe (32), die Kanüle und den Kanülenträger (10) umschließt und auf ihrer offenen Seite durch ein vom Benutzer abnehmbares Verschlussglied (71) verschlossen ist, so dass Abdeckkappe (66) und Verschlussglied (71) die erste Kappe (32), die Kanüle (12) und den Kanülenträger (10) steril umschließen, wobei die zweite Kappe (66) an ihrer zylindrischen Innenausnehmung drei Längsnuten (70) aufweist, welche gleichmäßig am Umfang der Innenausnehmung (68) verteilt und so bemessen sind, dass sie über drei Anschlagteile des Kanülenträgers (56, 56', 56") geschoben werden können.

2. Nadelanordnung nach Anspruch 1, bei welcher das vom Benutzer abnehmbare Verschlussglied als Peelfolie (71) ausgebildet ist.

3. Nadelanordnung nach einem oder mehreren der vorhergehenden Ansprüche, bei welcher auf der Außenseite (36) des Kanülenträgers (10) mindestens zwei Anschlagelemente (58, 60, 58', 60') zur Begrenzung der distalen Verschiebung der ersten Kappe (32) vorgesehen sind, welche jeweils durch eine Sollbruchstelle (76) mit dem Kanülenträger (10) verbunden sind,

4. Nadelanordnung nach Anspruch 3, bei welcher die Abdeckkappe (66) dazu ausgebildet ist, mindestens ein Anschlagelement (58, 60, 58', 60') des auf der Außenseite des Kanülenträgers (10) vorgesehenen Anschlags zu beeinflussen, um die Einstichtiefe (D) einzustellen.

5. Nadelanordnung nach Anspruch 4, bei welcher das mindestens eine Anschlagelement (58, 60) des auf der Außenseite des Kanülenträgers (10) vorgesehenen Anschlags über eine Sollbruchstelle (76) am Kanülenträger (10) befestigt ist, welche Sollbruchstelle (76) durch eine Drehbewegung (74) der mit diesem Anschlagelement in Eingriff gebrachten Abdeckkappe (66) abbrechbar ist.

6. Nadelanordnung nach einem der Ansprüche 3 bis 5, bei welcher die Sollbruchstelle (76) nach ihrem Bruch als axiale Führung für die Verschiebung der ersten Kappe (32) relativ zum Kanülenträger (10) dient.

7. Nadelanordnung nach einem oder mehreren der vorhergehenden Ansprüche, bei welcher die erste Kappe (32) auf einer im wesentlichen zylindrischen Umfangsfläche (36) des Kanülenträgers (10) verschiebbar angeordnet ist, und eine Verdrehsicherung (44,45) vorgesehen ist, weiche eine Verdrehung zwischen dem Kanülenträger (10) und der ersten Kappe (32) mindestens nahezu verhindert.

8. Nadelanordnung nach Anspruch 7, bei welcher die Verdrehsicherung (44, 45) mindestens eine Längsnut (44) aufweist, welche an erster Kappe (32) oder Kanülenträger (10) vorgesehen ist, und einen darin eingreifenden komplementären Vorsprung (45), welcher am entsprechenden Gegenstück, also Kanülenträger bzw. erster Kappe, vorgesehen ist.

9. Nadelanordnung nach einem oder mehreren der vorhergehenden Ansprüche, bei welcher die Feder als Kunststofffeder (26) ausgebildet ist.

10. Nadelanordnung nach Anspruch 9, bei welcher die Kunststofffeder (26) einstückig mit dem Kanülenträger (10) ausgebildet ist.

11. Nadelanordnung nach Anspruch 9 oder 10, bei welcher die Kunststofffeder (26) an ihrem proximalen Ende mit einem Ring (28) versehen ist, welcher gegen die erste Kappe (32) anliegt und diese in proximaler Richtung beaufschlagt.

12. Nadelanordnung nach Anspruch 11, bei welcher der Ring (28) einstückig mit der Kunststofffeder (26) ausgebildet ist.

13. Nadelanordnung nach einem oder mehreren der Ansprüche 9 bis 12, bei welcher die Kunststofffeder (26) zwei schraubenförmige Federelemente (26a, 26b) aufweist, von denen jedes mit dem Kanülenträger (10) einstückig ausgebildet ist.

## Claims

1. Needle arrangement for an injection apparatus (16), with an outer thread (18) provided at the proximal end of the injection apparatus (16), with a cannula carrier (10), on which a cannula (12) is fixed, and which is formed by an inner thread (20) on the cannula carrier (10) for detachable fixing on the injection apparatus (16), a first cap (32) displaceably arranged on the cannula carrier (10) roughly parallel to the longitudinal axis of the cannula (12), which at its proximal end section has a passage opening (42) for the cannula (12) and in its proximal end position essentially conceals the cannula (12), a compression spring (26) arranged between cannula carrier (10) and displaceable first cap (32) to move the first cap (32) to this proximal end position, wherein the needle arrangement comprises a second cap formed as a cover cap (66) and as a mounting aid for the screwing on of the needle arrangement onto the injection apparatus (16), enclosing the displaceable first cap (32), the cannula and the cannula carrier (10) and on its open side being sealed by a closing element (71) removable by the user, so that cover cap (66) and closing element (71) enclose the first cap (32), the cannula (12) and the cannula carrier (10) in a sterile way, wherein the second cap (66) carries on its cylindrical inner recess three longitudinal grooves (70) which are uniformly distributed on the periphery of the inner recess (68) and are dimensioned so that they can be pushed over three stop parts of the cannula carrier (56, 56', 56").

2. The needle arrangement according to claim 1, wherein the closing element is formed as peelable foil (71), removable by the user.

3. The needle arrangement according to one or more of the preceding claims, wherein at least two stops (58, 60, 58', 60') are provided on the outside (36) of the cannula carrier (10), each stop being connected by a predetermined breaking point (76) with the cannula carrier (10), said stops being provided for limiting the distal displacement of the first cap (32).

4. The needle arrangement according to claim 3, wherein the cover cap (66) is adapted to influence at least one stop (58, 60, 58', 60') of the abutment provided on the exterior of the cannula carrier (10) in order to set the penetration depth (D).

5. The needle arrangement according to claim 4, wherein the at least one stop (58, 60) of the abutment provided on the exterior of the cannula carrier (10) is fixed to the cannula carrier (10) via a predetermined breaking point (76), and said predetermined breaking point (76) is adapted to be broken off by a twisting motion (74) of the cover cap (66) being brought into engagement with this stop.

6. The needle arrangement according to any one of claims 3 to 5, wherein the predetermined breaking point (76) serves after its break as axial guidance for the displacement of the first cap (32) relative to the cannula carrier (10).

7. The needle arrangement according to one or more of the preceding claims, wherein the first cap (32) is displaceably arranged on an essentially cylindrical surface (36) of the cannula carrier (10), and a rotation prevention device (44, 45) is provided, which substantially prevents relative rotation between the cannula carrier (10) and the first cap (32).

8. The needle arrangement according to claim 7, wherein the rotation prevention device (44, 45) comprises at least one longitudinal groove (44), which is provided on the first cap (32) or the cannula carrier (10), and a complementary protrusion (45), engaging it, which is provided on the corresponding counterpart, that is to say the cannula carrier or the first cap.

9. The needle arrangement according to one or more of the preceding claims, wherein the spring is formed as a plastic spring (26).

10. The needle arrangement according to claim 9, wherein the plastic spring (26) is formed integrally with the cannula carrier (10).

11. The needle arrangement according to claim 9 or 10, wherein the plastic spring (26) at its proximal end is provided with a ring (28), which abuts against the first cap (32) and biases same in the proximal direction.

12. The needle arrangement according to claim 11, wherein the ring (28) is formed integrally with the plastic spring (26).

13. The needle arrangement according to one or more of claims 9 to 12, wherein the plastic spring (26) has two helical spring elements (26a, 26b), each of which is formed integrally with the cannula carrier (10).

## Revendications

1. Configuration d'aiguille pour un appareil pour injection (16) comportant un filetage extérieur (18) prévu sur l'extrémité proximale de l'appareil pour injection (16), un support de canule (10), sur lequel est fixée une canule (12) et qui est formé par un filetage intérieur (20) sur le support de canule pour être fixé de manière détachable à l'appareil pour injection (16), un premier capuchon (32) placé de manière coulissante sur le support de canule (10) à peu prés parallèlement à l'extension longitudinale de la canule (12), le capuchon ayant au niveau de sa section terminale proximale une ouverture de passage (42) pour la canule (12) et recouvrant en grande partie la canule (12) dans sa position terminale proximale, un ressort de compression (26) placé entre le support de canule (10) et le premier capuchon (32) coulissant pour faire coulisser le premier capuchon (32) dans cette position terminale proximale, la configuration d'aiguille comportant un deuxième capuchon constituant un couvercle (66) et une aide au montage pour le vissage de la configuration d'aiguille sur l'appareil pour injection (16), qui recouvre le premier capuchon (32) coulissant, la canule et le support de canule (10) et qui est fermé sur son côté ouvert par un élément de fermeture (71) que l'utilisateur peut retirer, de sorte que le couvercle (66) et l'élément de fermeture (71) recouvrent de manière stérile le premier capuchon (32), la canule (12) et le support de canule (10), le second capuchon (66) présentant sur son évidement intérieur cylindrique trois rainures longitudinales (70), qui sont réparties uniformément sur le pourtour de l'évidement intérieur (68) et sont dimensionnées de telle sorte qu'elles peuvent être glissées au moyen de trois parties de butée du support de canule (56, 56', 56").

2. Configuration d'aiguille selon la revendication 1, dans laquelle l'élément de fermeture détachable par l'utilisateur est une feuille pelable (71).

3. Configuration d'aiguille selon une ou plusieurs des revendications précédentes, dans laquelle sur le côté externe (36) du support de canule (10) sont prévus au moins deux éléments de butée (58, 60, 58', 60') pour la limitation du déplacement distal du premier capuchon (32), chacun étant relié par un emplacement destiné à la rupture (76) au support de canule (10).

4. Configuration d'aiguille selon la revendication 3, dans laquelle le couvercle (66) est formé afin d'influencer au moins un élément de butée (58, 60, 58', 60') de la butée prévue sur le côté externe du support de canule (10), afin d'établir la profondeur de piqûre (D).

5. Configuration d'aiguille selon la revendication 4, dans laquelle le au moins un élément de butée (58, 60) de la butée prévue sur le côté externe du support de canule (10) est fixé sur le support de canule (10) par un emplacement destiné à la rupture (76), ledit emplacement destiné à la rupture (76) pouvant être rompu par un mouvement de rotation (74) du couvercle (66) qui est mis en engrènement avec cet élément de butée.

6. Configuration d'aiguille selon l'une des revendications 3 à 5, dans laquelle l'emplacement destiné à la rupture (76) après sa rupture sert au guidage axial pour le déplacement du premier capuchin (32) par rapport au support de canule (10).

7. Configuration d'aiguille selon une ou plusieurs des revendications précédentes, dans laquelle le premier capuchon (32) est placé de manière coulissante sur une surface périphérique dans l'ensemble cylindrique (36) du support de canule (10) et un limiteur de rotation (44, 45) est prévu, qui empêche au moins à peu de chose près une rotation entre le support de canule (10) et le premier capuchon (32).

8. Configuration d'aiguille selon la revendication 7, dans laquelle le limiteur de rotation (44, 45) présente au moins une rainure longitudinale (44) qui est prévue sur le premier capuchon (32) ou le support de canule (10), et une saillie complémentaire (45) engrenant avec la rainure, saillie prévue sur l'élément opposé correspondant, c'est-à-dire le support de canule ou le premier capuchon.

9. Configuration d'aiguille selon une ou plusieurs des revendications précédentes, dans laquelle le resort est formé comme ressort en plastique (26).

10. Configuration d'aiguille selon la revendication 9, dans laquelle le ressort en plastique (26) est realize d'une seule pièce avec le support de canule (10).

11. Configuration d'aiguille selon la revendication 9 ou 10, dans laquelle le ressort en plastique (26) est doté à son extrémité proximale d'un anneau (28) qui 9 10 prend appui contre le premier capuchon (32) et qui charge celui-ci en direction proximale.

12. Configuration d'aiguille selon la revendication 11, dans laquelle l'anneau (28) est réalisé d'une seule pièce avec le ressort en plastique (26).

13. Configuration d'aiguille selon une ou plusieurs des revendications 9 à 12, dans laquelle le ressort en plastique (26) présente deux éléments de resort (26a, 26b) en forme d'hélice, chacun étant realize d'une seule pièce avec le support de canule (10).
